# EUROPÄISCHE PATENTSCHRIFT

(11) **EP 2 661 230 B1**
(45) Veröffentlichungstag und Bekanntmachung des Hinweises auf die Patenterteilung: **23.03.2016**
(21) Anmeldenummer: 12700096.6
(22) Anmeldetag: 03.01.2012
(51) Int. Cl.: A61B 17/02

(54) **RETRAKTIONSSYSTEM**
RETRACTION SYSTEM
SYSTÈME ÉCARTEUR

(30) Priorität: 03.01.2011 DE 102011002412
(43) Veröffentlichungstag der Anmeldung: 13.11.2013
(73) Patentinhaber: Salomon, Dirk, 04626 Jonaswalde (DE); Mühlhäusler, Bernd, 06507 Bad Suderode (DE); Fischer, Christian, 06507 Bad Suderode (DE)
(72) Erfinder: Salomon, Dirk, 04626 Jonaswalde (DE)
(74) Vertreter: Lippert, Stachow & Partner
(86) Internationale Anmeldenummer: PCT/EP2012/050045
(87) Internationale Veröffentlichungsnummer: WO 2012/093114

(56) Entgegenhaltungen:
- WO-A1-94/06354
- GB-A- 1 520 832
- US-A- 2 493 598
- US-A- 2 695 607
- US-A- 4 421 107
- US-A- 6 117 072
- US-A1- 2008 021 286
- US-A1- 2010 030 260
- US-B1- 6 824 511

## Beschreibung

Die Erfindung betrifft allgemein ein Retraktionssystem zum Darstellen von Zugängen in der Human, Tier- oder Dentalmedizin.

Zum Offenhalten von chirurgischen Zugängen werden verschiedene Retraktionsinstrumente.verwendet, bei denen Retraktorelemente, z.B. Wundhaken, in die Inzision eingesetzt werden, um mittels Zugkraft einen Operationszugang herzustellen. Als Retraktorelemente sind beispielsweise Hohmann-Haken bekannt, welche die gegenüberliegenden Seiten des eröffneten Zugangs auseinanderziehen. Problematisch dabei ist es vor allem, die ständige Fixierung des Patienten im ausreichenden Maße zu gewährleisten. Dazu wird mindestens ein Assistent benötigt, welcher die Öffnung mittels der Hohmann-Haken auseinanderzieht.

Ein weiteres Problem ergibt sich bei lang anhaltenden Eingriffen dadurch, dass mit zunehmender Zeitdauer das Risiko unwillkürlicher Bewegungen durch den Assistenten zunimmt, was wiederum eine erneute Fixierung erfordert oder eventuell auch Auswirkungen auf den Operationsverlauf haben könnte. Ein weiterer wesentlicher Nachteil ergibt sich zudem daraus, dass für den Operateur mitunter die Zugänglichkeit zum Operationsfeld sowie Sicht und Beleuchtung eingeschränkt sind, was den OP-Ablauf wesentlich stört.

In der US 2008/0021286 A1 wird ein Retraktor beschrieben, für dessen Anwendung zwar kein zusätzliches Personal erforderlich ist. Jedoch wird auch hier der chirurgische Zugang durch die beschriebene Anordnung zumindest teilweise verdeckt. Die zweiteilige Form basiert auf diesem Dokument.

Aus diesem Grund sind bereits verschiedene, zum Teil sehr aufwendig anzusetzende Haltesysteme bekannt, die in Verbindung mit den Retraktorelementen ein Retraktionssystem bilden. Bekannt sind sehr komplexe Retraktionssysteme, bei denen mittels verschiedener Gestänge die Retraktorelemente gehalten und fixiert werden. Diese sind jedoch nicht in jedem Fall einsetzbar und schränken mitunter das Operationsfeld unerwünscht ein.

Die WO 94/06354 A1 beschreibt ein Retraktionssystem für Gliedmaßen, welches aus zwei Bändern besteht, die an ihren einem Ende jeweils einen Haken zur Retraktion aufweisen. Die beiden Bänder umfassen dabei das Körperglied derart, dass je ein Band ausgehend von der Inzision zirkulär um das Körperglied herumgeführt wird. Auf der Unterseite des Körperglieds stehen die beiden Bänder über eine Klemmvorrichtung im Eingriff miteinander. Dadurch wird eine zirkulär wirkende Zugkraft bewirkt, welche das geöffnete Gewebe spreizen, wobei eine stufenweise Anpassung der Zugkraft mittels der Klemmvorrichtung erfolgt.

In der US 6,117,072 werden Retraktorhaken beschrieben, die an einen kreisförmigen Rahmen befestigt werden können, wodurch ein Hineinfallen in den chirurgischen Zugang verhindert werden kann.

Ein Nachteil einiger vorbeschriebener Retraktionssysteme besteht darin, dass durch die Verwendung von metallischen Werkstoffen das intraoperative Röntgen erheblich erschwert wird, was insbesondere im Operationsbereich unerwünscht ist.

Weiterhin müssen die vorbeschriebenen Retraktoren unter der Operation entnommen und wieder eingesetzt werden, z.B. um intraoperative Röntgenaufnahmen oder Bewegungsmanipulationen am Körperglied zuzulassen. Danach ist regelmäßig eine erneute Fixierung erforderlich. Dadurch verlängern sich zum einen die Operationszeiten und es erhöht sich auch die Gefahr einer Infektion für den Patienten. Des Weiteren kann auch das Gewebe bei wiederholter Einsetzung geschädigt werden.

Zudem müssen die bekannten Systeme nach ihrer Verwendung einem Reinigungs- und Sterilisationsprozess unterzogen werden, um für eine weitere Operation eingesetzt werden zu können. Auch dadurch besteht ein Infektionsrestrisiko bei Verwendung derartiger Systeme, da hierbei eventuelle Probleme auftreten können. Zudem besteht bei aufwändig ausgestalteten Systemen das Problem, das gesamte System zu Sterilisieren, was aufgrund der Gestalt und häufig auch der Größe problematisch ist.

Die Aufgabe der vorliegenden Erfindung besteht daher darin ein Retraktionssystem anzugeben, welches adaptierbar für die verschiedensten Operationsfelder einsetzbar ist und die vorbeschriebenen Nachteile überwindet.

Die Aufgabe wird durch ein Retraktionssystem gemäß dem Hauptanspruch gelöst. Vorteilhafte Ausgestaltungen sind in den abhängigen Ansprüchen angegeben.

Erfindungsgemäß wird ein am jeweiligen Körperabschnitt angeordnetes Retraktionssystem mit zumindest einem hakenförmigen Retraktorelement und Haltemittel zu dessen Fixierung am Körperabschnitt vorgeschlagen welches aus einem röntgentransparenten Material ausgeführt ist und selbsthaltend oder zumindest abschnittsweise biegsam ausgebildet ist oder beide letztgenannten Eigenschaften aufweist. Die Biegsamkeit ist dergestalt, dass sich ein biegsames Element oder ein biegsamer Abschnitt mit der Retraktion anschmiegend an ein zu retrahierendes Gewebe verhält.

Das vorgeschlagene Retraktionssystem gestattet sowohl in Verbindung mit der selbsthaltenden Fixierung am Patienten als auch mit der biegsamen Eigenschaft die Verwendung von röntgentransparenten Materialien, so dass während einer Operation Röntgenuntersuchungen durchführbar sind. Die durch das erfindungsgemäße Retraktionssystem gegebene Fixierung des Operationsfeldes kann während des Röntgens unter der Operation weiterhin erfolgen, weshalb eine sonst notwendige Neufixierung entfällt.

Als selbsthaltend ist hier zu verstehen, dass die Stabilisierung des verwirklichten Retraktionssystems und die für die Retraktion aktuell aufzubringenden Haltekräfte ohne externen Eingriff realisiert werden und dies in situ am Körper entsprechend der für das Anwendungsgebiet allgemeinen und subjektiv besonderen Gegebenheiten. Die vom Retraktionssystem aufgenommenen Kräfte sind hinreichend groß, um die zur Retraktion erforderlichen Zugkräfte zu übernehmen und das System in sich zu stabilisieren. Dementsprechend kann ein derart autark wirkendes Retraktionssystem sowohl an die Anforderungen der verschiedenen Körperteile als auch die besonderen körperlichen Bedingungen eines Patienten sowie der operativen Erfordernisse angepasst werden. Darüber hinaus kann die Befestigung am zu behandelnden Körperteil den anatomischen Gegebenheiten in situ angepasst erfolgen.

Die selbsthaltende Funktionsweise gestattet eine durch das Körperteil und die Operationsbedingungen, nicht oder in geringem Umfang durch das Retraktionssystem eingeschränkte Bewegungsfreiheit des Körperteils, so dass z.B. Mobilitätstests im Retraktionszustand erfolgen können.

Die anatomische Anpassung an das jeweilige Körperteil und dessen Maße, so dass das Retraktionssystem selbsthaltend ist, kann auf verschiedene Weise geschehen. In vielen Fällen kann das Körperteil zirkulär umgriffen werden, wobei die Haltekräfte, die durch das System selbst aufgebracht werden, auch aufgrund einer sich aus der zirkulären Umfassung ergebenden radialen Kraftkomponente verstärkt werden können. Ergänzend sind gemäß besonderer Ausgestaltungen auch Verbindungen zu angrenzenden Körperteilen möglich. Darüber hinaus gestattet das Retraktionssystem auch Verknüpfungen zu externen Konstruktionen, um ein Zusammenwirken in solchen Fällen zu erzielen, in denen die Art des Eingriffs dies erfordert.

Ergänzend zur selbsthaltenden Eigenschaft ist das Retraktionssystem zumindest abschnittsweise biegsam ausgebildet und zwar in der Art, dass zumindest das betreffende Retraktorelement und je nach verwendetem Funktionselement auch dessen Teile sich mit der Retraktion an ein zu retrahierendes Gewebe anschmiegen. Die Verbindung des röntgentransparenten Materials mit der selbsthaltenden und/oder biegsamen Eigenschaften verbessert die Handhabbarkeit auch im Verlauf der Operation, indem das Retraktionssystem mehr dem Körperteil zugeordnet wird als die bestehenden Systeme.

Durch eine geeignete Form der betreffenden Elemente und Abschnitte können diese in situ an das Operationsfeld und die anatomischen Gegebenheiten selbst anschmiegend eingesetzt werden. In Verbindung mit der Verwendung von röntgentransparentem Material können die biegsamen Eigenschaften an den jeweiligen Anwendungsfall und die damit verbundene Kräftesituation sehr gezielt angepasst werden. Dies kann durch die Wahl des Materials und/oder die Gestalt des Elements oder Abschnittes so erfolgen, dass das gewünschte Anschmiegen im Operationsfeld und eine selbst optimierende Retraktion für unterschiedliche Operationsfelder, z.B. hinsichtlich der Größe und des zu spreizenden Gewebes, und Patienten, z.B. kräftige und weniger kräftige, erzielbar ist.

Die biegsame, anschmiegende Eigenschaft der besagten Elemente bewirkt im Vergleich zu den bekannten starren Retraktoren zudem ein verbessertes Öffnen des Operationsfeldes. Neben dem Spreizen des Gewebes wird durch nachgebende und sich anschmiegende Elemente gleichzeitig das umgebende Gewebe niedergehalten. Je nach Einsatz der Retraktorelemente können zudem auch Körperteile im Operationsfeld besser positioniert und/oder fixiert, wie z.B. Knochen angehoben, werden. Im Ergebnis kann der Zugang für den Operateur deutlich verbessert werden.

Die anschmiegende Eigenschaft und die Anpassungsfähigkeit des Retraktionssystems an die jeweilige Situation bewirkt des Weiteren, dass Stress für Gewebe vermieden werden kann. Indem durch die biegsamen Elemente Belastungen, die auf das Gewebe einwirken, vermittelt werden, können z.B. Quetschungen oder Zerrungen verhindert werden.

Entsprechend einer Ausgestaltung ist das Retraktionssystem modular aus verschiedenen miteinander in verschiedenster Weise kombinierbaren Elementen aufgebaut. Es umfasst Funktionselemente, die auf sehr unterschiedliche Weise miteinander kombinierbar sind und so Anpassungen an das Operationsfeld für die verschiedensten Körperteile gestatten und die notwendige Retraktion übernehmen. Es umfasst außerdem Schloss- und Schließelemente, welche die Verbindung der Funktionselemente miteinander selbsthaltend realisieren. Dazu weist jedes Funktionselement zumindest ein Schloss- oder Schließelement auf. Dieses grundlegende System aus Funktionselementen und Schloss- und Schließelementen, von denen jedes Funktionselement zumindest eines aufweist, gestattet eine Minimierung der Einzelteile bei größtmöglicher Variabilität des Systems entsprechend den in großem Umfang variierenden anatomischen Gegebenheiten.

Die wesentlichen Elemente eines Retraktionssystems sind folglich Retraktorelemente als grundlegende Funktionselemente, welche die Öffnung des Zugangs sicherstellen, sowie Schlosselemente und Schließelemente, wobei als Schließelement das Gegenstück zum Schlosselement bezeichnet sein soll, welches direkt in das Schloss eingreift. Zumindest die Schlosselemente, gegebenenfalls auch beide sind selbsthaltend ausgeführt und ermöglichen eine kraftschlüssige Verbindung verschiedener Elemente des Gesamtsystems untereinander, z.B. der Retraktorelemente untereinander oder zu anderen Elementen.

Die Verbindung von Retraktorelementen mit Schloss- oder Schließelementen führt, wie am Ausführungsbeispiel unten näher dargestellt, dazu, dass eine Verstellbarkeit bei der Montage eines Retraktorelements in dessen Wirkrichtung, d.h. in Retraktionsrichtung besteht. Dies verbessert die Einstellbarkeit der Retraktion in situ und den anatomischen Gegebenheiten entsprechend deutlich, insbesondere in Verbindung mit einem modularen zusammengesetzten Retraktionssystem.

Unterstützt durch das modulare System können als Retraktorelemente neben den üblichen bekannten Retraktoren wie Hohmann ähnliche Haken, Langenbeck ähnliche Haken in allen Längen und Breiten, scharfe, spitze, stumpfe Haken sowie Roux-Haken auch Haken verwendet werden, die in ihrer Form und Wirkungsweise für spezielle Anwendungen ausgelegt sind. Die Retraktorelemente können z.B. auch mit Beleuchtungshilfen ausgestattet sein.

Weitere Funktionselemente des modular konzipierten Gesamtsystems können sein:
- Verlängerungselemente, welche nach Bedarf unter der Operation eine umfangreichere Längenanpassung des Retraktionssystems ermöglichen,
- Querverbindungselemente, welche zur Stabilisation und Lagefixierung zwischen benachbart angeordneten Funktionselementen, insbesondere benachbarten Retraktorelmenten eingesetzt werden,

- Ausgleichselemente, welche einen Winkelausgleich bzw. Längenausgleich bei nicht symmetrischer Eröffnung des Zugangs ermöglichen,
- spezifische Formelemente, welche als anatomisch angepasste Funktionselemente eine Anpassung des Retraktionssystems an die anatomischen Gegebenheiten im Bereich des Zugangs ermöglichen, sowie
- Adaptionselemente, welche eine Kombination mit anderen Instrumenten und Vorrichtungen sicherstellen.

Weitere Funktionselemente sind integrierbar. Mittels der integrierten und/oder separaten Schloss- und Schließelemente sind die Funktionselemente zur Realisierung verschiedener Funktionen miteinander kombinierbar.

So weisen Querverbindungselemente, die zur Stabilisation und Lagefixierung zwischen benachbart angeordneten Retraktoren eingesetzt werden, Schloss- und/oder Schließelemente in verschiedenen möglichen Kombinationen auf, wie oben zu den Verlängerungselementen beschrieben. Dies ist insbesondere vorteilhaft, um eine koordinierte Retraktion mit gemeinsamer Zugrichtung zwischen benachbarten Retraktoren zu gewährleisten.

Ausgleichselemente werden verwendet, um eine optimale geometrische Anpassung des gesamten Systems oder einzelner Komponenten in Winkel, etwa bei Einsatz des Retraktionssystems im Gelenksbereich, und Länge, etwa mittels elastischer Funktionselemente, zu erreichen. Dadurch wird eine optimale Anpassung des Retraktionssystems an den Operationsbereich gewährleistet, was eine verbesserte Retraktionswirkung bei verringerter Belastung des Patienten ermöglicht. Auch deren Verbindung zu angrenzenden Funktionselementen des Retraktionssystems kann über Schloss- und Schließelement erfolgen.

Spezifische, der Anatomie oder dem Zugang angepasste Formelemente werden verwendet, um die vorbeschriebenen Retraktionssysteme zusätzlich zu unterstützten. Dabei sind schalenartige Funktionselemente, wie etwa eine Handgelenksauflage oder auch separate Halteelemente, wie etwa Vorrichtungen, welche auf den Finger aufgesteckt werden, vorgesehen. Dies ist insbesondere vorteilhaft um eine Fixierung angrenzender Gliedmaßen zu bewirken oder diese in die Haltevorrichtung des Retraktionssystems zu integrieren. Sofern es die Gestaltung der Formelemente gestattet, kann auch deren Verbindung zu angrenzenden Funktionselementen des Retraktionssystems über Schloss- und Schließelemente alternativ unter Einfügung z.B. eines Ausgleichs- oder eines Verlängerungselements erfolgen.

Wie oben benannt sind Adaptionselemente vorgesehen, mit deren Hilfe das Retraktionssystem zum Halten oder Führen von anderen Instrumenten, wie Vorrichtungen zum Absaugen, endoskopischen Instrumenten, Sonden, Infusionsschläuchen, Röntgenmarkierungen, Optiken, Beleuchtungsmitteln, Navigationshilfen, Führungen oder Lehren bzw. sonstigen Gerätschaften genutzt oder mit anderen Fixationssystemen gekoppelt wird. Dabei wird das erfindungsgemäße Retraktionssystem mittels Adaptionselementen genutzt, um als Haltevorrichtung für weitere Instrumente zu fungieren. Dadurch lässt sich eine gezielte Führung der Instrumente im Operationsbereich sicherstellen, was letztlich eine verbesserte Zugänglichkeit des eröffneten Operationsbereichs ermöglicht. Auch die Adaptionselemente können optional über Schloss- und Schließelemente mit dem System verbunden sein.

Ein weiterer Vorteil des modularen Retraktionssystems ergibt sich dadurch, dass im Gegensatz zu bekannten Systemen zwischen sterilen und unsterilen Elementen getrennt werden kann, da nur diejenigen Elemente des Systems steril zu halten sind, welche in Kontakt mit dem Operationsfeld stehen. Dies werden regelmäßig zumindest die Retraktorelemente sein. Andere, wie z.B. Adaptions- oder Verlängerungselemente und die zugehörigen oder zuzuornnenden Schloss- und Schließelemente können im unsterilen Bereich liegen. Eine Trennung zwischen sterilen und unsterilen Elementen ist entsprechend dem Operationsbereich nahezu frei wählbar. Die Komplettierung des Systems kann somit sowohl zeitlich als auch hinsichtlich der Sterilität und einer möglichen Verknüpfung mit weiterem Operationsequipment flexibel den Anforderungen angepasst werden.

Sofern Schließ-, Schloss- oder verschiedene Funktionselemente biegsam ausgebildet sind, ergeben sich zudem bessere Verbindungsmöglichkeiten der verschiedenen Elemente während der OP, was insbesondere für ein modulares Retraktionssystem vorteilhaft ist.

In einer weiteren Ausgestaltung der Erfindung sind die zuvor benannten biegsamen Elemente oder Elementabschnitte elastisch ausgebildet. Die elastische Eigenschaften eines oder mehrerer Elemente oder Abschnitte davon führen dazu, dass die besagten Elemente und Abschnitte nach der Entlastung wieder ihre Ausgangsform einnehmen und eine Vorspannung aufweisen können, die die für die Retraktion erforderlichen Kräfte sowie auch das selbsthaltende System unterstützt. Hinzu kommt, dass eine Vorspannung eines elastischen Elements nutzbar ist, die Anpassung an die Kräftesituation im OP-Feld und damit die Zugänglichkeit zu den frei zu legenden Körperteilen wie oben dargelegt weiter zu verbessern.

Welche Elemente die biegsamen oder optional elastischen Eigenschaften aufweisen sollten, hängt wesentlich vom Operationsfeld ab. Je nachdem, welche der oben genannten, neben dem Spreizen weiteren möglichen Effekte erzielt werden sollen, können das entweder allein ein oder mehrere Retraktorelemente oder ein oder mehrere Funktionselemente bzw. Abschnitte davon oder auch verschiedene Kombinationen davon sein. Sind die Retraktorelemente biegsam bzw. elastisch, ergeben sich bei ihrer Anwendung Formen, die gut an die Kräftesituation gegenüber den Geweben und Knochen angepasst sind. Es ergeben sich Haken- und/oder Hebelformen, die verbesserte Retraktionseffekte erzielen. Bei einem modular aufgebauten Retraktionssystem kann die Anpassung des Retraktionssystems an die gegebene Situation aktuell vorgenommen werden.

In einer weiteren Ausführungsform der Erfindung weisen Funktionselemente des Retraktionssystems, insbesondere die Retraktorelemente bandartige Enden auf, die als Schließelement dienen und durch ein selbsthaltendes Schlossteil kraftschlüssig verbunden werden.

Die bandartigen Enden gestatten in Verbindung mit den Schlosselementen eine einfache Handhabung unter Operationsbedingungen, verschiedene Verbindungsmöglichkeiten zu Schlosselementen und zudem strukturell einfache Konstruktionen, die auch Vereinfachungen hinsichtlich der Sterilisierung ermöglichen. Zudem gestatten die bandförmigen Enden entsprechend einer Ausgestaltung der Erfindung die stufenlose Längeneinstellung über einen frei wählbaren, großen Bereich. Die stufenlose Einstellung kann z.B. über ein Klemmen des Bandes im Schlosselement erzielt werden.

Die Schloss- und Schließelemente sind wie oben beschrieben selbsthaltend gestaltet, wobei verschiedene unten im Detail beschriebene Bauformen möglich sind. Die unten folgenden Beschreibungen von Schlosselementen mit stegartigen Klemmelementen, welche die eingefügten Schließelemente festklemmen, sollen lediglich beispielhaft solche kraftschlüssige Verbindungen zwischen Schloss- und Schließelementen zeigen, mit denen eine stufenlose Verstellung beider Funktionselemente zueinander gewährleistet ist.

In einer weiteren Ausführungsform der Erfindung sind Schlosselemente und/oder Schließelemente separate Teile zur Kopplung von mehreren anderen Funktionselementen. Dies ist insbesondere vorteilhaft um eine flexible Gestaltung des Retraktionssystems entsprechend den anatomischen Bedingungen im Operationsbereich zu gewährleisten.

Um die Positionierung der Verbindung von Funktionselementen zu vereinfachen oder für eine schnelle Komplettierung unter der Operation vorzubereiten, kann ein Schließelement ein Positionierungsmsittel aufweisen, mittels dessen ein separates Schlosselement positionierbar ist.

Alternativ oder ergänzend sind Schlosselemente und/oder Schließelemente funktionell einstückig mit Retraktorelementen verbunden oder in andere Funktionselemente integriert, so dass ein Retraktorelement oder ein anderes Funktionselement Schloss- und/oder Schließelemente in verschiedener Anzahl und Anordnung aufweisen kann. Dies ist vorteilhaft, um eine schnelle Fixierung und Lösung der verschiedenen Elemente zu gewährleisten. So ist es auch möglich Elemente mit funktionell fest oder auch standardmäßig integrierten Schloss- und/oder Schließelementen bei solchen Eingriffen zu verwenden, wo die anatomischen Bedingungen des Operationsbereiches nur geringe Abweichungen zeigen.

Z.B. können Verlängerungselemente, welche in der Regel bandartig ausgestaltet sind, ein oder zwei Schlosselemente oder Schließelemente aufweisen. Auch die Kombination von einem oder mehreren Schloss- und Schließelementen an einem Verlängerungselement ist möglich. Die Schloss- und Schließelemente sind dabei so ausgebildet und miteinander kombiniert, dass Verlängerungselemente für alle der oben genannten Funktionselemente des Retraktionssystems zur Verfügung stehen. Vorteilhafterweise werden die Verlängerungselemente vor allem bei stark adipösen Patienten verwendet.

Aufgrund der verschiedenen Kombinationsmöglichkeiten der Funktionselemente ist es von Vorteil, wenn in einer Ausgestaltung des Retraktionssystems Schlosselemente zwei oder mehr Schließelemente aufmehmen können. Damit können mehrere Funktionselemente an einem Punkt zusammenlaufen und ohne Einschränkung der Funktionalität die Elementeanzahl optimiert werden. So sind auch winklige Verbindungen entsprechend der anatomischen Gegebenheiten in einem Winkelbereich zwischen 0 und 180° zwischen dem Schloss- und einem Schließelement oder zwei oder mehr im Schlosselement zusammenlaufenden Schließelementen möglich.

Um in Abhängigkeit von der Gestaltung der Schließelemente ein einfaches und schnelles Verbinden mit einem Schlosselement zu ermöglichen, können in verschiedenen Ausgestaltungen die Schlosselemente unterschiedliche Verbindungsmittel aufweisen. So sind Mittel zum Einstecken des Schließelements möglich, wodurch einfach Längeneinstellungen über die Tiefe des Einsteckens eines bandförmigen Schließelements über einen großen Bereich möglich. Auch ein Vorbereiten der Verbindung ist möglich, indem das Schließelement zunächst lose eingesteckt und erst nach der endgültigen Positionierung des zu haltenden Funktionselements endgültig verbunden wird.

Auch Mittel zum Einlegen sind möglich, die es gestatten, zuerst das Funktionselement ordnungsgemäß zu positionieren und dann ohne Notwendigkeit der Verschiebung zu fixieren. Hierbei oder in anderen Verbindungsarten kann ein Einschnappen des Schließelements in einer Vor- oder endgültigen Position hilfreich sein, um die Positionierung oder Fixierung akustisch durch ein Schnappgeräusch zu signalisieren.

Bei den benannten Verbindungsmöglichkeiten ist es von Vorteil, wenn zumindest ein Schließelement aus einem elastischen Material besteht. Insbesondere für eine anatomische Anpassung der Funktionselemente kann es darüber, hinaus von Vorteil sein, wenn auch die Schließelemente und gegebenenfalls auch Schlosselemente elastisch verformbar sind.

In einer weiteren Ausführungsform weist die Oberfläche des röntgentransparenten Materials zumindest in den Abschnitten, in welchen die Verbindung der Elemente des Retraktionssystems, d.h. der Schloss- und Schließ- sowie der Funktionselemente erfolgt, eine Mikrostrukturierung auf, welche die selbsthaltende Wirkung des Retraktionssystems verstärkt. Die Mikrostrukturierung der Oberfläche des röntgentransparenten Materials ist als eine erhöhte Oberflächenrauhigkeit zu verstehen, welche zu einer Verbesserung der Haltewirkung des Retraktionssystems führt.

Die Strukturen können in Abhängigkeit vom verwendeten Material durch das Material selbst bedingt sein, z.B. bei der Verwendung von strukturierten Geweben, oder durch Bearbeitung oder Beschichtung der Oberflächen der betreffenden Abschnitte hergestellt sein. Alternativ kann auch der Herstellungsprozess der Elemente, z.B. deren Formgebung derart konzipiert werden, dass die fertigen Elemente die gewünschte Oberflächenstruktur aufweist.

Zur Unterstützung der stufenlosen Einstellbarkeit weisen die Oberflächenstrukturen Größenordnungen im Bereich bis maximal 2 mm, vorzugsweise im Bereich von 0,1 mm bis 1 mm auf und können regelmäßig, z.B. als Oberflächentextur oder stochastisch ausgebildet sein.

Aus der Verwendung von röntgenstrahlendurchlässigem Material ergeben sich weitere Vorteile. Wird z.B. ein hochtemperaturbeständiger, thermoplastischer Kunststoff wie beispielsweise der Hochleistungskunststoff Polyetheretherketon (PEEK) oder PE verwendet, kann dieser neben der thermischen und mechanischen Festigkeit auch die oben benannte elastisch Verformbarkeit aufweisen zur Selbstadaptierung der Elemente an anatomische Verhältnisse.

Ein weiterer Vorteil der Verwendung von Kunststoff besteht in der Möglichkeit, das Retraktionssystem, zumindest jedoch die steril zu haltenden Elemente als Einwegelemente auszuführen. Hierdurch entfällt die sonst notwendige Sterilisation vor Beginn der Operation, was wiederum zu einer Verkürzung des Zeitaufwands zur Operationsvorbereitung führt, da diese bereits nach Herstellung sterilisiert und verpackt werden können.

Nachfolgend soll die Erfindung anhand von Figuren und Ausführungsbeispielen eingehender erläutert werden. Es zeigen
- Fig. 1: eine schematische Darstellung einzelner Bestandteile eines erfindungsgemäßen Retraktionssystems,
- Fig. 2A und 2B: eine Ausgestaltung eines Retraktionssystems mit elastischen Elementen in zwei verschiedenen Einsatzzuständen an einem Gliedmaß (Schnittdarstellung),
- Fig. 3A bis 3E: Ausgestaltungen von Schloss- und Schließelementen,
- Fig. 4A, 4B: Schnittdarstellungen von Schloss- mit Schließelementen,
- Fig. 5A bis 5E: Ausgestaltungen von Schlosselementen, die in Funktionselementen integriert sind,
- Fig. 6: eine Ausgestaltung eines Schlosselements mit einer gekreuzten Steckung,
- Fig. 7A, 7B: eine schematische Darstellung von Ausgestaltungen von einschnappbaren Schließelementen an einem bandartigen Ende,
- Fig. 8: ein Schloss- mit Schließelement mit Mikrostrukturierung der Oberfläche des Schließelements, und
- Fig. 9: eine schematische Darstellung eines beispielhaften erfindungsgemäßen Retraktionssystems für den Handbereich.

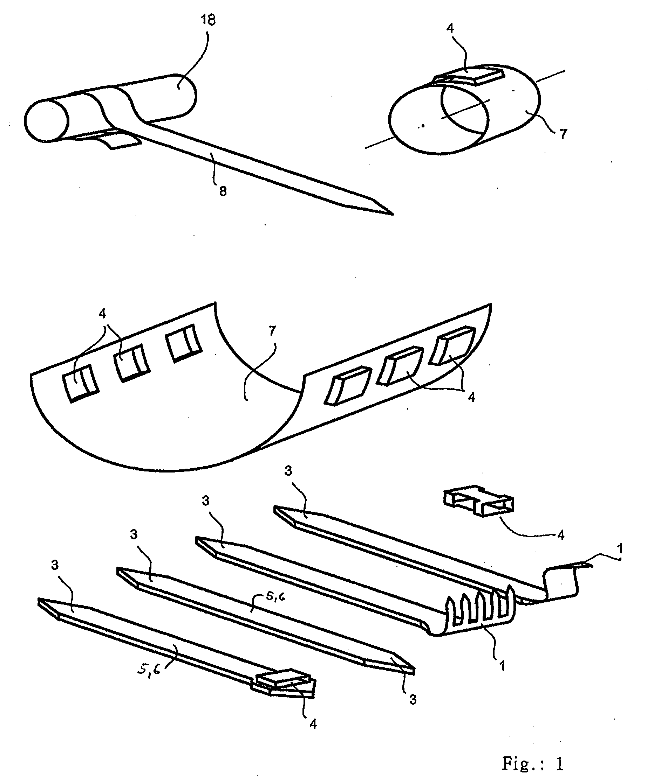

In der Fig. 1 sind zunächst beispielhaft einzelne Bestandteile des Retraktionssystems dargestellt.

Zur Retraktion des chirurgischen Zugangs werden Retraktorelemente 1 verwendet, deren Form an die Anforderungen im Operationsfeld angepasst ist. Dabei können Retraktorelemente 1 wie Hohmann ähnliche Haken, Langenbeck ähnliche Haken in allen Längen und Breiten, scharfe, spitze, stumpfe Haken, Roux-Haken sowie auch Haken und andere Formen verwendet, die in Ihrer Form und Wirkungsweise für spezielle Anwendungen ausgelegt sind. Die Retraktorelemente 1 sind in den dargestellten Ausführungsformen beispielhaft jeweils mit einem Schließelement 3 verbunden, indem sie an einem Ende eines bandförmigen Elements angeordnet sind. Die anderen Enden des Bandes dienen als Schließelemente 3. Auch separate Retraktorelemente 1 sind möglich, die auf geeignete Weise mit einem der weiteren Funktionelemente verbindbar sind.

Weiterhin umfasst das Retraktionssystem Schlosselemente 4, welche entweder an einem Ende eines bandförmigen Funktionselements angeordnet sind oder wie in der Fig. 1 dargestellt als separates Element ausgestaltet. Die Schlosselemente 4 sind selbstklemmend ausgeführt und dienen der kraftschlüssigen Verbindung mit den Schließelementen 3. Dabei werden, wie unten im Detail beschrieben wird, die Schließelemente 3 in das Schlosselement 4 eingeführt, z.B. eingesteckt, wodurch sich die selbstklemmende Wirkung der Schlosselemente 4 ergibt. Eine stufenlose Retraktion ist durch Einstecken der Schließelemente 3, welche am Ende der Retraktorelemente 1 angeordnet sind, in die Schlosselemente 4 möglich. Z.B. ergibt sich bei zirkulärem Umgreifen eines Körperelements je nach dessen Größe meist eine radiale Kraftkomponente vom Schließelement 3 auf das Schlosselement 4, welche die Klemmwirkung in lateraler Richtung verstärkt.

Das in Fig. 1 dargestellte Retraktionssystem umfasst weiterhin Verlängerungselemente 5, welche vorzugsweise bandförmig ausgestaltet sind. Die Verlängerungselemente 5 sind insbesondere bei Verwendung des Retraktionssystems an Gliedmaßen oder Körperregionen mit erhöhtem Umfang vorteilhaft, welche durch die an den Retraktorelementen 1 angeordneten Bänder nicht umfasst werden können. Die Verlängerungselemente 5 können hierbei ebenfalls Schlosselemente 4 und Schließelemente 3 aufweisen, um eine selbstklemmende Verbindung zwischen den einzelnen Elementen zu gewährleisten, was wiederum die Zugkraft für die Retraktion durch einfaches Einstellen der stufenlosen Verbindung ermöglicht. Das Verlängerungselement 5 gemäß Fig. 1 weist zwei Schließelemente 3 in Form bandförmiger Enden auf.

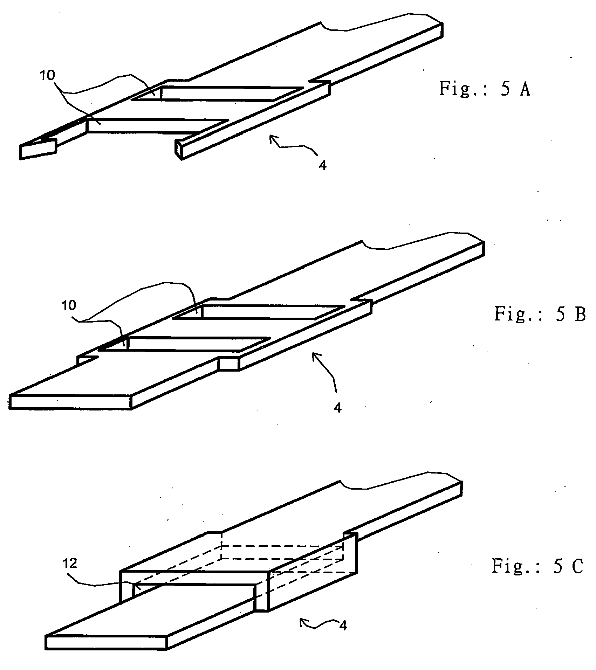

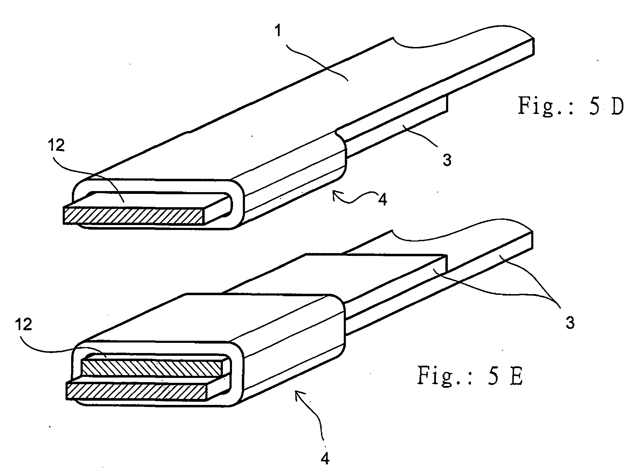

Zur weiteren Stabilisierung eines Retraktionssystems sind weiterhin Querverbindungselemente 6 vorgesehen, die eine Verbindung zwischen den einzelnen Retraktorelementen 1 ermöglichen und somit zu einer verbesserten Fixierung der Retraktorelemente 1 führen. Die Querverbindungselement 6 sind vergleichbar den Verlängerungselementen 5 vorzugsweise bandförmig ausgestaltet. Die Querverbindungselemente 6 und Verlängerungselemente 5 sind in Fig. 1 mit den verschieden möglichen Schloss- und Schließelementen 3, 4 ausgestattet. Sie sind als Band ausgeführt und weisen an einem Ende ein bandförmiges Schließelement 3 und am anderen ein Schlosselement 4 oder an beiden Enden Schließelemente 3 auf. Fig. 1 zeigt des Weiteren eine mögliche Form eines separaten Schlosselements 4. Es wird näher in Fig. 5 dargestellt und beschrieben.

Neben den bisher beschriebenen Elementen umfasst das Retraktionssystem weiterhin spezifische, der Anatomie oder dem Zugang angepasste Formelemente 7. Diese Formelemente 7 werden entsprechend den vorliegenden anatomischen Gegebenheiten an ein Gliedmaß, etc. angeordnet. Beispielsweise kann ein Formelement 7 als Funktionselement zum Aufstecken auf einen Finger, wie in Fig. 1 dargestellt, ausgeführt sein. Oder es ist aus einer größeren, verschieden gestaltbaren Platte geformt, zum Anlegen an flächige Körperteile, z.B. ein Gliedmaß oder den Rücken. In jedem Fall weist auch ein Formelement 7 ein Schließ- oder ein Schlosselement 3, 4 auf, welches die Aufnahme eines zusätzlichen Retraktorelements 1 oder die Verbindung mit einem weiteren Funktionselement ermöglicht. Alternativ können auch andere Funktionselemente, welche über eine Schließelement 3 verfügen an dem spezifischen anatomischen Formelement 7 angeordnet werden, wie etwa Beleuchtungsmittel, etc.

Weiterhin umfasst das Retraktionssystem Adaptionselemente 8, welche zum Halten und Führen von anderen Instrumenten, wie Sonden, Absaugvorrichtungen, endoskopischen Instrumenten, etc. verwendet werden. Alternativ können die Adaptionselemente 8 zur Verbindung mit anderen Fixationssystemen als externes System 18, d.h. dem Retraktionssystem nicht zugehörig, vorgesehen sein. Durch die Verwendung von Adaptionselementen 8 ist eine erweiterter modularer Aufbau des Retraktionssystems und damit eine spezifische Anpassung an die Bedürfnisse des Operateurs möglich. Die verschiedenen Funktionselemente gemäß Fig. 1 können aufgrund der Tatsache, dass jedes der Funktionselemente zumindest ein Schlosselement 4 oder ein Schließelement 3 aufweist, in verschiedensten Kombinationen miteinander verbunden werden.

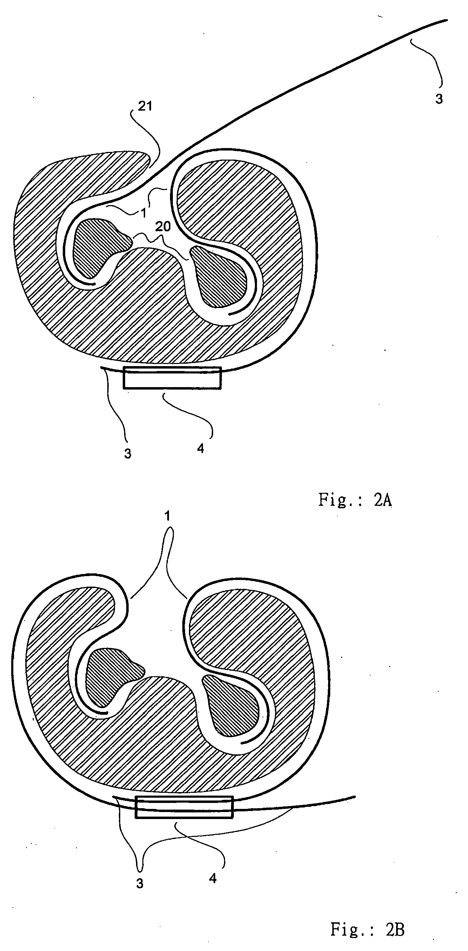

In der Fig. 2A ist beispielhaft eine schematische Darstellung eines erfindungsgemäßen Retraktionssystems wiedergegeben. Dabei erfolgt eine Retraktion des chirurgischen Zugangs mittels zweier Retraktorelemente 1, deren Form an einem Ende jedes bandförmigen Retraktorelements 1 den bekannten Hakenformen nachempfunden ist. Die Retraktorelemente 1 und die sich daran anschließenden bandförmigen Verlängerungen sind bis zu den Schließelementen 3 elastisch ausgeführt, wobei die vorgebogenen, hakenförmigen Enden der Retraktorelemente 1 eine solche Elastizität aufweisen, die es gestattet unter Beibehaltung ihrer Form höhere Kräfte aufnehmen zu können. Sofern erforderlich, können sie auch starr ausgebildet sein. Das dargestellte Retraktionssystem umfasst neben den elastischen Retraktor- mit Schließelementen 1, 3 ein separates Schlosselement 4, in das beide Schließelemente 3 in entgegengesetzte Richtungen eingeschoben werden können.

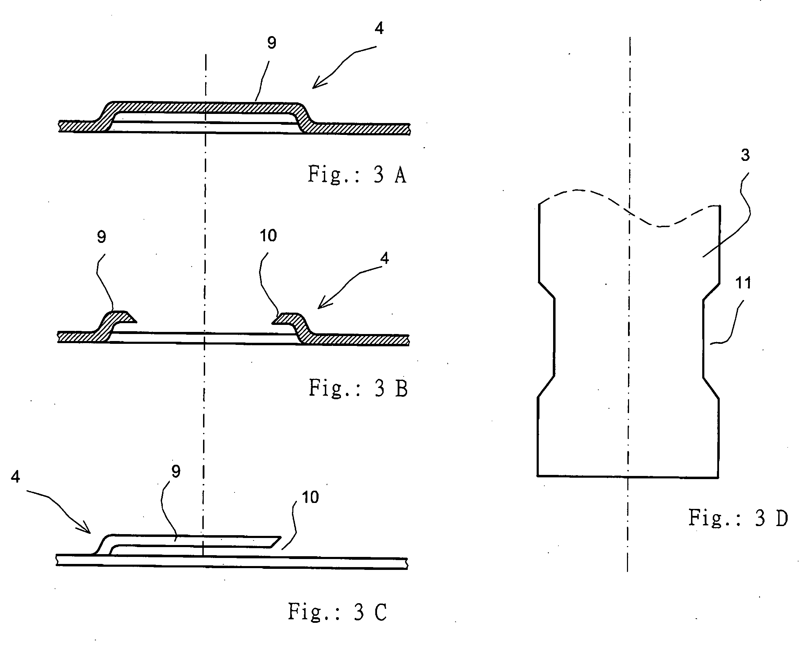

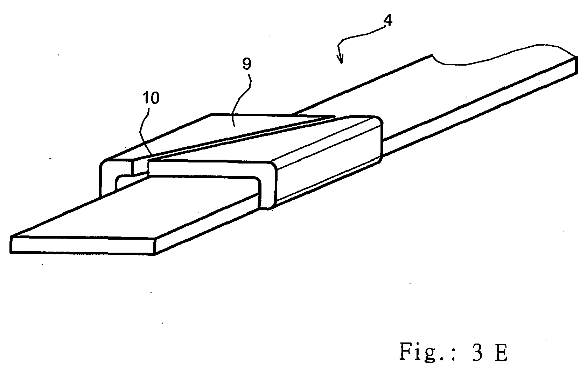

Die Verbindungen zwischen den einzelnen Retraktorelementen 1 der Retraktionssysteme erfolgt wie oben beschrieben mittels des Schlosselements 4 und der Schließelemente 3. Alternativ zur dargestellten Ausführung können diese auch an einem Retraktorelement 1 miteinander kombiniert vorliegen. Dafür sind die zu den Fig. 3 bis Fig. 8 dargestellten und unten näher beschriebenen verschiedenen Bauformen möglich.

Fig. 2A stellt das Retraktionssystem während dessen Positionierung dar, wobei bereits einer der beiden Schließelemente 3 mit dem Schlosselement 4 verbunden ist. Die bandförmige Verlängerung des in Fig. 2A rechten Retraktorelements 1, welches an seinem freien Ende ein Schließelement 3 aufweist, umfasst dabei halbumfänglich ein zu operierendes Gliedmaß, so dass das Schließelement 3 mit dem Schlosselement 4 in Eingriff steht. Durch Einstecken des Schließelements 3 in das Schlosselement 4 erfolgt eine selbstklemmende Verbindung zwischen Schloss- und Schließelement 3, 4.

Das linke Retraktionselement 1 ist ebenfalls unter dem Knochen 20 positioniert. Dessen Schließelement 3 ist jedoch noch nicht mit dem Schlosselement 4 verbunden, so dass lediglich dessen vorgeformtes Ende eine der im endgültigen Einsatzzustand ähnliche Form aufweist. Die bandförmige Verlängerung umgreift im dargestellten Zustand das Gewebe 21 noch nicht, so dass dessen formgebende Wirkung auf das Gewebe im Vergleich zum rechten, eingesetzten Element sichtbar wird.

Aufgrund der Elastizität der vorgeformten freien Enden der Retraktorelemente 1 sind diese gut unter die Knochen 20 einzusetzen. Die Elastizität der übrigen Abschnitte gestattet ein einfaches Verbinden mit dem Schlosselement 4.

In Fig. 2B ist auch das Schließelement 3 des linken, zum rechten baugleichen Retraktor- mit Funktionselement 1 mit dem Schlosselement 4 verbunden, so dass sich die beiden Schließelemente 3 überlappen. Die Retraktion erfolgt nunmehr durch stufenlose Anpassung der Zugkraft der Retraktorelemente 1 über die Längenänderung der Schließelemente 3 in den Schlosselementen 4. An diesem Beispiel ist die Übereinstimmung von Einstell- mit Retraktionsrichtung erkennbar. Während der Retraktion schmiegen sich die elastischen bandartigen Teile der Funktionselemente an die Gewebeform an und formen dieses, die Kräfte ausgleichend, leicht nach.

Aufgrund der Elastizität der freien Enden der Retraktorelemente 1 wird bei deren Einsetzen unter die frei gelegten Knochen 20 über das darüber liegende, von dem Funktionselement umfasste Gewebe 21 eine Hebelwirkung auf die Knochen 20 ausgeübt , so dass die Knochen 20 leicht in Richtung Öffnung angehoben werden (nicht dargestellt). Gleichzeitig wird das umgebende Gewebe 21 durch die sich an Knochen 20 und Gewebe 21 anschmiegenden Retraktorelemente 1 gespreizt, zur Seite gehalten und dabei leicht niedergedrückt. Im Ergebnis ermöglichen die elastischen Retraktorelemente 1 einen guten Zugang z.B. zum Knochen 20.

Die folgenden Ausgestaltungen zeigen verschiedene Schloss- und Schließelemente 3, 4, die für ein modulares Retraktionssystem verwendbar sind. In einer Ausgestaltung gemäß Fig. 3A weist ein Schlosselement 4 ein stegartiges Klemmelement 9 in jenem Bereich auf, der im Einsatzfall vorzugsweise auf der dem Körper abgewandten Seite des Schlosselements 4 liegt, nachfolgend als oberer Bereich bezeichnet. In das Klemmelement 9 kann ein dazu passendes Schließelement 3, z.B. ein bandartiges Ende eines anderen Funktionselements (nicht dargestellt) eingesteckt werden, so dass es kraftschlüssig gehalten wird.

Alternativ kann in einem Schlosselement 4 das stegartige Klemmelement 9 abschnittsweise ausgespart sein, so dass ein Schließelement 3 (nicht dargestellt) eingeschnappt werden kann. Fig. 3B zeigt eine Aussparung 10 an der Längsseite des Steges des Klemmelements 9.

In einer weiteren Ausgestaltung eines Schlosselements 4 weist das stegartige Klemmelement 9 eine seitliche Aussparung 10 auf (Fig. 3C), durch welche ein Schließelement 3 (nicht dargestellt) eingeschoben und eingerastet werden kann und durch die im Einsatzfall angreifenden Kräfte in seiner Position gehalten wird.

Die Einführung des Schließelements 3 in eine Aussparung 10 des stegartigen Klemmelements 9 kann durch eine passende Gestalt des Schließelements 3 unterstützt werden. In Fig. 3D ist beispielhaft eine abschnittsweise Verjüngung 11 des bandartigen Schließelements 3 dargestellt. Nach dessen Einführung durch die Aussparung 10 kann das Schließelement 3 bis zur korrekten Position verschoben und dort fixiert werden.

In einer weiteren Ausgestaltung eines Schlosselements 4 weist das stegartige Klemmelement 9 eine schräge Aussparung 10 auf (Fig. 3E) auf, durch welche ein Schließelement 3 (nicht dargestellt) eingelegt, gegebenenfalls zur Längeneinstellung verschoben werden kann und durch die im Einsatzfall angreifenden Kräfte in seiner Position gehalten wird.

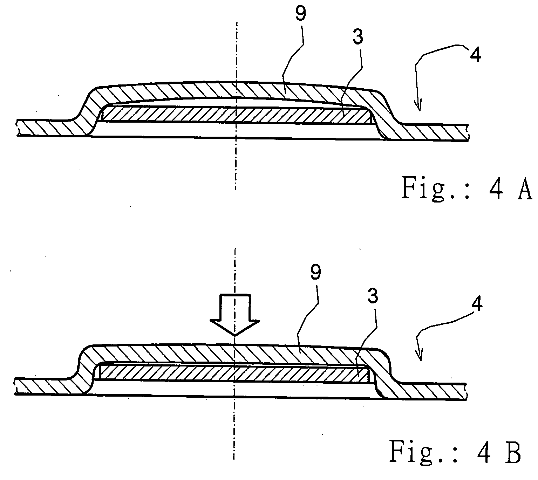

Eine alternative Ausführung der Schlosselemente 4 als Klemmelemente 9 gestattet eine einfache Lösung der Verbindung zwischen Schloss- 4 und Schließelement 3, wie in Fig. 4A dargestellt. Indem Kraft auf den Steg des Klemmelements 9 ausgeübt wird, in Fig. 4B durch Pfeile in deren Wirkungsrichtung dargestellt, verbreitert sich der durch den Steg gebildete Durchgang, so dass die seitlichen Ränder des Schließelements 3, z.B. eines bandförmigen Endes eines Retraktorelements 1, freigegeben werden (Fig. 4B). In dieser Position lässt sich das Schließelement 3 verschieben und durch Beenden der Krafteinwirkung wieder festklemmen. Eine solche Wirkungsweise ist einfach anwendbar und wird insbesondere durch die Herstellung der Elemente aus Kunststoff, Gewebe oder dergleichen als einteilige, gut zu sterilisierende und preiswert herzustellende Komponenten unterstützt.

In den Fig. 5A bis Fig. 5E sind verschiedene Ausgestaltungen des Schlosselements 4 dargestellt, welche an einem der Funktionselemente angeordnet sind, wodurch eine stufenlos einstellbare Verbindung mit anderen Funktionselementen durch Einfügen von deren Schließelemente 3 herstellbar ist.

Die Ausführungen gemäß Fig. 5A und 5B sind vergleichbar einer Schnalle ausgeführt und sind für elastisch verformbare bandförmige Schließelemente 3 (nicht dargestellt) geeignet. Während in der Ausführung nach Fig. 5A das Schlosselement am Ende eines zumindest in diesem Abschnitt bandförmigen Funktionselements angeordnet ist, befindet es sich in Fig. 5B im mittleren Abschnitt eines zumindest abschnittsweise bandförmigen Funktionselements. Die Aufnahme eines ebenfalls bandförmigen Schließelements 3 erfolgt über die beiden nebeneinander angeordneten Aussparungen 10.

Das Schlosselement 4 gemäß Fig. 5C ist durch einen Höhensprung in einem bandförmigen Abschnitt eines Funktionselements gebildet, mit einem parallel zur Bandoberfläche verlaufenden Durchgang 12 in einem sich überlappenden Teil des Bandes.

Das an einem bandförmigen Ende eines ersten Funktionselements, z.B. eines Retraktorelements 1 ausgebildete Schlosselement 4 gemäß Fig. 5D ist geeignet ein bandförmiges Ende eines weiteren Funktionselements, z.B. eines weiteren Retraktorelements 1 oder eines Verlängerungselements 5, als dessen Schließelement 3 so aufzunehmen, dass beide Funktionselemente in der gleichen Richtung verlaufen, z.B. für eine Verlängerung eines Retraktorelements 1 oder für die direkte Verbindung zweier aufeinanderzulaufender Retraktorelemente 1 analog jener in Fig. 2. Dafür ist das Schlosselement 4 am Ende eines Funktionselements eine parallel zur Bandoberfläche und in Richtung des Bandes verlaufenden Durchgang 12 auf. Auch hier sind alternativ andere Winkel zwischen Durchgang und Band möglich.

Alternativ kann der Durchgang 12 in einem separaten Schlosselement 4 ausgebildet sein und eine Durchgangshöhe aufweisen, die das Einschieben und Klemmen von zwei parallel verlaufenden Schließelementen 3 (Fig. 5E) oder mehreren gestattet.

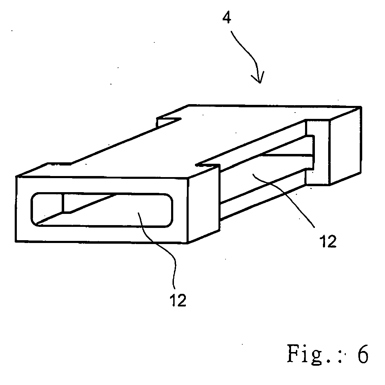

In dem Ausführungsbeispiel nach Fig. 6 ist das Schlosselement z.B. als separates Teil derart ausgeführt, dass es die Aufnahme von zwei Schließelementen 3 ermöglicht, vergleichbar der Ausführung in Fig. 5E. Die dargestellte Ausgestaltung ermöglicht jedoch die Querverbindung einzelner Funktionselemente. Dafür weist das Schlosselement 4 wie in Fig. 6 dargestellt einen ersten Durchgang 12 als Aufnahmemöglichkeit für ein Schließelement 3 (nicht dargestellt) und einen zweiten Durchgang 12 als Aufnahmemöglichkeit für ein weiteres Schließelement 3 (nicht dargestellt) auf, wobei der zweite Durchgang 12 in einem zum ersten Durchgang 12 abweichenden Winkel angeordnet ist. In der Fig. 6 ist beispielhaft eine orthogonale Anordnung der beiden Durchgänge 12 für Schließelemente 3 dargestellt. Davon abweichend kann der Winkel entsprechend den Bedürfnissen im Operationsfeld ausgestaltet sein und zwischen 0° und 180° liegen.

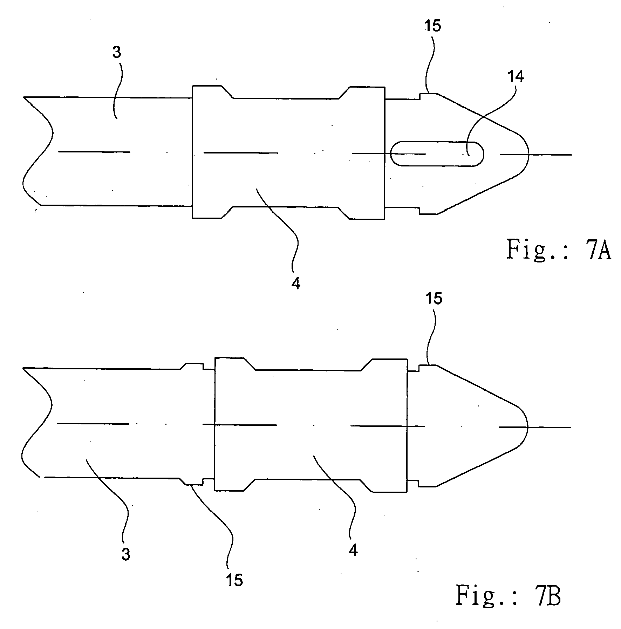

In Fig. 7A und 7B sind alternative Ausgestaltungen von Schließelementen 3 dargestellt, die z.B. an einem Verlängerungselement 5 oder einem Querverbindungselement 6 oder anderen angeordnet sein können. Das bandförmige Schließelement 3 weist an seinem Ende eine flächige Verdickung 15 des Bandes auf, die mit einem Schlitz 14 auf der Bandlängsachse korrespondiert. Aufgrund des Schlitzes 14 kann das Schließelement 3 in ein Schlosselement 4, hier beispielhaft eines gemäß Fig. 6, gezwängt werden, bis es hinter der Verdickung 15 einschnappt. Auf diese Weise sind Verbindungen von verschiedenen Funktionselementen vorzubereiten und zu sichern, um im Moment des Einsatzes einfach und schnell auf die entsprechende Länge festzustellen. Das in Fig. 7A verwendete Schlosselement 4 realisiert die Verbindung mit einem weiteren Funktionselement entlang der Bandlängsachse des Schließelements 3, welches das Schlosselement 4 trägt, oder senkrecht dazu.

Die Ausführung gemäß Fig. 7B unterscheidet sich von der gemäß Fig. 7A durch das Fehlen des Schlitzes und durch die Ergänzung einer weiteren Verdickung 15, die spiegelbildlich und mit einem solchen Abstand zur ersten am Bandende ausgeführt ist, dass ein über die erste Verdickung 15 gezwängtes Schlosselement 4 nur geringfügig und zwar bis zur zweiten Verdickung 15 auf dem Band verschoben werden kann und ein Zwängen über die zweite Verdickung 15 nicht oder nur sehr schwer möglich ist. Auch damit sind Verbindungen zwischen Funktionselementen für den späteren Einsatz vorbereitbar, gemäß Fig. 7B jedoch auf eine definierte Lage des Schlosselements 4 beschränkt. Auch in Fig. 7B ist ein Schlosselement gemäß Fig. 6 dargestellt. Alternativ sind auch andere, separat oder an einem Funktionselement ausgebildet einsetzbar.

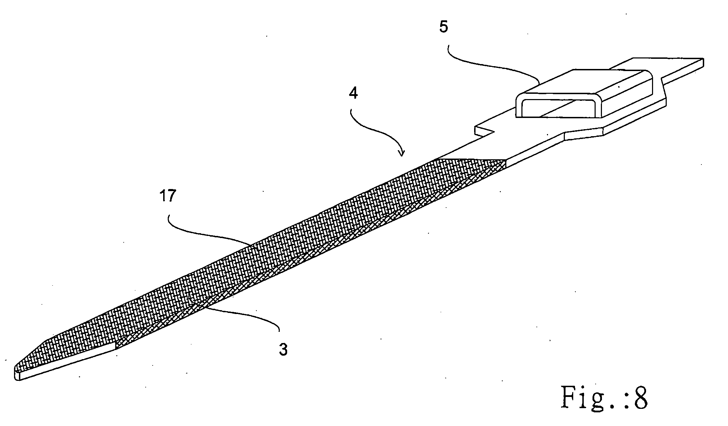

Das in Fig. 8 dargestellte Funktionselement, z.B. ein bandförmiges Verlängerungselement 5, an dessen einem Ende ein Schließelement 3 und am anderen Ende ein Schlosselement 4 z.B. gemäß Fig. 3A ausgebildet ist. Zur Verstärkung der selbstklemmenden Haltekräfte weist das Schließelement 3 an seiner Oberfläche eine Mikrostrukturierung 17 auf. Diese ist z.B. als Textur, d.h. aus regelmäßig wiederkehrenden geometrischen Strukturen in der Größenordnung von einigen Zehntel Millimetern ausgebildet. Alternativ sind auch unregelmäßige Strukturen verwendbar, die z.B. mittels Strahlverfahren herstellbar sind. Alternativ oder ergänzend kann eine Mikrostrukturierung auch an der Kontaktfläche eines Schlosselements 4 ausgebildet sein, mit dem Schließelement 3 kraftschlüssig in Verbindung steht.

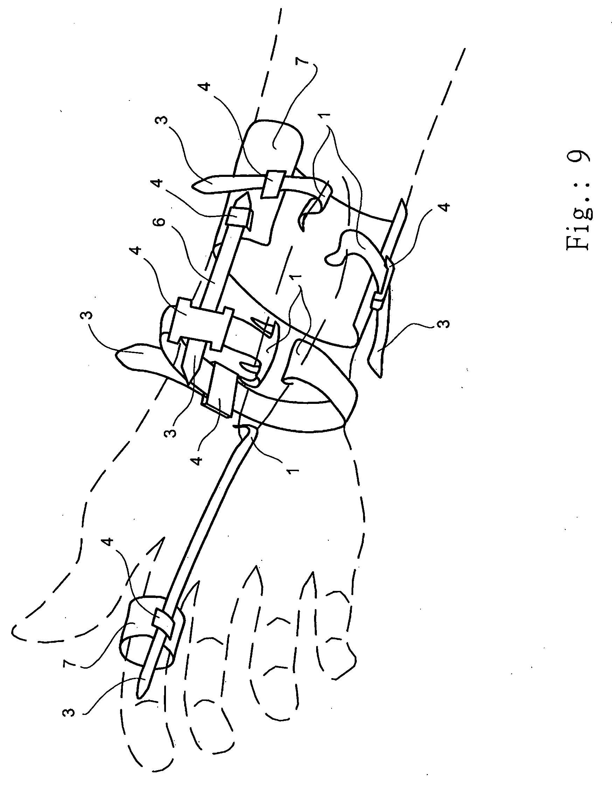

In einem weiteren Ausführungsbeispiel ist in der Fig. 9 beispielhaft ein erfindungsgemäßes Retraktionssystem zur Verwendung im Handgelenksbereich schematisch dargestellt. Das Retraktionssystem gemäß Fig. 9 umgreift das Körperteil, hier ein Handgelenk in zwei beabstandeten Lagen zirkulär und weist zur Retraktion und zur Fixierung des gesamten Systems eine Reihe unterschiedlicher Funktionselemente auf.

Das System umfasst dabei ein plattenförmiges Formelement 7, welches an der gegenüberliegenden Seite des zu eröffnenden Bereichs des Handgelenks angeordnet ist und sich elastisch dem Teilumfang des Handgelenks anpasst. Die Retraktion erfolgt über vier Retraktorelemente 1, welche unterschiedlich ausgestaltet sein können. Am Ende der Retraktorelemente 1 sind Schließelemente 3 in Form von bandförmigen Enden angeordnet, eine für das Umgreifen des jeweiligen Körperabschnitts erforderliche Längeneinstellung gestattet. Andererseits ist es dem Operateur zudem leicht möglich die Längeneinstellung jederzeit zu korrigieren oder entsprechend dem Operationsverlauf anzupassen.

Die bandförmigen Schließelemente 3 der beiden Retraktorelemente 1 der ersten Retraktorlage sind in jeweils ein Schlosselement 4 des Formelements 7 eingesteckt und werden durch eine Klemmwirkung, wie zu Fig. 4A beschrieben festgeklemmt. Dadurch erfolgt eine selbstklemmende, stufenlose Retraktion.

Neben diesen beiden Retraktorelementen 1 ist zudem ein Querverbindungselement 6 mit dem Formelement 7 verbunden, welches eine stabile Querverbindung zwischen den zwei Retraktorlagen realisiert. Dabei ist ein bandförmiges Schließelement 3 des Querverbindungselements 6 in einem weiteren Schlosselement 4 des Formelements 7 gehalten. Dieses Schlosselement 4 ist rechtwinklich zu den beiden zuvor beschriebenen Schlosselementen 4 angeordnet, so dass das Querverbindungselement 6 längs des Handgelenks angeordnet ist. Am anderen Ende des Querverbindungselements 6 erfolgt eine Verbindung mit einem Retraktorelement 1 über ein separates Schlosselement 4 mit orthogonaler Aufnahmemöglichkeit des Schließelements 3 des Querverbindungselements 6. Das Schlosselement 4 ist dabei analog dem in Fig. 5 ausgestaltet.

Das separate Schlosselement 4 nimmt in der zweiten Ebene das Band eines Retraktorelements 1 der zweiten Retraktorlage auf, so dass auch diese Retraktorlage rechtwinklig zum Querverbindungselement 6 liegt. Zur zweiten Retraktorlage gehört ein weiteres Retraktorelement 1. Die beiden Retraktorelemente 1 der zweiten Lage sind über ein Schlosselement 4 und ein Schließelement 3 miteinander verbunden, von denen jedes in je einem der beiden Retraktorelemente 1 integriert ist.

Weiterhin umfasst das Retraktionssystem ein spezifisches, anatomisches Formelement 7 in Form eines Fingeraufsatzes. Diese ringartige Formelement 7 ist auf einen Finger gesteckt und weist dabei ein Schlosselement 4 zur Aufnahme eines Schließelements 3 eines weiteren, in Richtung Handgelenk verlaufenden Retraktorelements 1 auf.

Auch in dieser Ausgestaltung können verschiedene Funktionselemente, Abschnitte davon oder auch Schließelemente 3 elastisch ausgebildet sein. Eine Elastizität z.B. der das Handgelenk zirkulär umgreifenden und gegebenenfalls auch bis zum Finger verlaufenden Retraktorelemente 1 sowie des Formelements 7 gestatten die Adaption des dargestellten Systems an verschiedene Größen und Stärken eines Handgelenks.

### Bezugszeichenliste

- 1: Retraktorelement
- 3: Schließelement
- 4: Schlosselement
- 5: Verlängerungselement
- 6: Querverbindungselement
- 7: Formelement
- 8: Adaptionselement
- 9: stegartiges Klemmelement
- 10: Aussparung
- 11: Verjüngung
- 12: Durchgang
- 13: Ausgleichselemente
- 14: Schlitz
- 15: Verdickung
- 17: Mikrostrukturierung
- 18: externes System
- 20: Knochen
- 21: Gewebe

## Patentansprüche

1. Retraktionssystem zum Darstellen von Zugängen in der Human, Tier- oder Dentalmedizin, wobei dieses selbsthaltend ausgeführt ist, am jeweiligen Körperabschnitt angeordnet wird und ein hakenförmiges Retraktorelement (1) und Haltemittel zu dessen Fixierung am Körperabschnitt umfasst, wobei das Retraktionssystem aus einem röntgentransparenten Material ausgeführt ist, **dadurch gekennzeichnet, dass** das Retraktionssystem zumindest abschnittsweise biegsam ausgebildet ist, so dass sich ein biegsames Element oder ein biegsamer Abschnitt bei einer Retraktion anschmiegend an ein zu retrahierendes Gewebe verhält.

2. Retraktionssystem nach Anspruch 1, **dadurch gekennzeichnet, dass** das Retraktionssystem modular ausgebildet ist, indem es Funktionselemente umfasst, die durch Schloss- und Schließelemente (3, 4) miteinander verbindbar sind, wobei jedes Funktionselement zumindest ein Schlosselement (4) oder ein Schließelement (3) als Haltemittel aufweist.

3. Retraktionssystem nach Anspruch 1 oder 2, **dadurch gekennzeichnet, dass** ein Funktionselement zumindest abschnittsweise biegsam ausgebildet ist.

4. Retraktionssystem nach einem der Ansprüche 1 bis 3, **dadurch gekennzeichnet, dass** ein Retraktorelement (1) biegsam ausgebildet ist.

5. Retraktionssystem nach einem der Ansprüche 1 bis 4, **dadurch gekennzeichnet, dass** das biegsame Funktionselement oder der biegsame Abschnitt elastisch ist.

6. Retraktionssystem nach Anspruch 2 bis 5, **dadurch gekennzeichnet, dass** mittels eines Schließelements (3) die Retraktion in Richtung ihrer Wirkung verstellbar ist.

7. Retraktionssystem nach Anspruch 2 bis 6, **dadurch gekennzeichnet, dass** ein Schließelement (3) als bandartiges Ende ausgebildet ist, welches in ein Schlosselement (4) eingreift.

8. Retraktionssystem nach einem der Ansprüche 2 bis 7, **dadurch gekennzeichnet, dass** die Schloss- und Schließelemente (3, 4) stufenlos zueinander verstellbar sind.

9. Retraktionssystem nach einem der Ansprüche 2 bis 8, **dadurch gekennzeichnet, dass** ein Schlosselement (4) und/oder ein Schließelement (3) als separates Teil ausgeführt sind.

10. Retraktionssystem nach Anspruch 9, **dadurch gekennzeichnet, dass** ein Schließelement (3) ein Positionierungsmittel aufweist zur Positionierung eines separaten Schlosselements (4).

11. Retraktionssystem nach einem der Ansprüche 2 bis 10, **dadurch gekennzeichnet, dass** ein Schlosselement (4) derart ausgebildet ist, dass es mehrere Schließelemente (3) hält.

12. Retraktionssystem nach einem der Ansprüche 2 bis 11, **dadurch gekennzeichnet, dass** ein Schlosselement (4) derart ausgebildet ist, dass es zwei Schließelemente (3) oder ein Schließelement (3) mit einem Funktionselement in einem Winkel im Bereich zwischen 0 und 180° miteinander verbindet.

13. Retraktionssystem nach einem der Ansprüche 2 bis 12, **dadurch gekennzeichnet, dass** ein Schlosselement (4) Mittel aufweist zum Einstecken, und/oder zum Einlegen und/oder zum Einschnappen des Schließelements.

14. Retraktionssystem nach einem der Ansprüche 2 bis 13, **dadurch gekennzeichnet, dass** ein Schließelement (3) des Retraktionssystems aus einem elastischen Material bestehen.

15. Retraktionssystem nach einem der Ansprüche 2 bis 14, **dadurch gekennzeichnet, dass** die Oberfläche eines Schließ- und/oder Schlosselements (3, 4) zumindest abschnittsweise eine die selbsthaltende Kraft verstärkende Mikrostrukturierung (17) aufweist.

## Claims

1. Retraction system for exposing accesses in human medicine, veterinary medicine or dentistry, wherein said retraction system is made to be self-retaining, is arranged on the respective body section and comprises a hook-shaped retractor element (1) and holding means for fixing it on the body section, wherein the retraction system is made of a material transparent to x-rays, **characterized in that** the retraction system is embodied to be flexible, at least in sections, such that, during a retraction, a flexible element or a flexible section nestles against tissue to be retracted.

2. Retraction system according to Claim 1, **characterized in that** the retraction system has a modular design by virtue of comprising functional elements that can be connected to one another by lock elements and closing elements (3, 4), wherein each functional element has at least one lock element (4) or one closing element (3) as holding means.

3. Retraction system according to Claim 1 or 2, **characterized in that** a functional element is embodied to be flexible, at least in sections.

4. Retraction system according to one of Claims 1 to 3, **characterized in that** a retractor element (1) is embodied to be flexible.

5. Retraction system according to one of Claims 1 to 4, **characterized in that** the flexible functional element or the flexible section is elastic.

6. Retraction system according to Claim 2 to 5, **characterized in that** the retraction can be adjusted in terms of the direction of its effect by means of a closing element (3).

7. Retraction system according to Claim 2 to 6, **characterized in that** a closing element (3) is embodied as tape-like end, which engages into a lock element (4).

8. Retraction system according to one of Claims 2 to 7, **characterized in that** the lock elements and closing elements (3, 4) can be adjusted continuously with respect to one another.

9. Retraction system according to one of Claims 2 to 8, **characterized in that** a lock element (4) and/or a closing element (3) are configured as a separate part.

10. Retraction system according to Claim 9, **characterized in that** a closing element (3) has a positioning means for positioning a separate lock element (4).

11. Retraction system according to one of Claims 2 to 10, **characterized in that** a lock element (4) is embodied in such a way that it holds a plurality of closing elements (3).

12. Retraction system according to one of Claims 2 to 11, **characterized in that** a lock element (4) is embodied in such a way that it connects two closing elements (3) or a closing element (3) and a functional element to one another at an angle in the range between 0 and 180°.

13. Retraction system according to one of Claims 2 to 12, **characterized in that** a lock element (4) has means for inserting and/or laying-in and/or snapping-in the closing element.

14. Retraction system according to one of Claims 2 to 13, **characterized in that** a closing element (3) of the retraction system consists of an elastic material.

15. Retraction system according to one of Claims 2 to 14, **characterized in that** the surface of a closing element and/or lock element (3, 4) has, at least in sections, microstructuring (17) which amplifies the self-retaining force.

## Revendications

1. Système de rétraction pour exposer des accès dans la médecine humaine, animale ou dentaire, lequel est réalisé de manière autoportante, est disposé au niveau de la portion du corps respective et comprend un élément rétracteur (1) en forme de crochet et des moyens de fixation pour sa fixation à la portion de corps, le système de rétraction étant réalisé à partir d'un matériau transparent aux rayons X, **caractérisé en ce que** le système de rétraction est réalisé sous forme au moins en partie flexible, de telle sorte qu'un élément flexible ou une partie flexible tienne de manière adaptée étroitement à un tissu à retirer lors d'une rétraction.

2. Système de rétraction selon la revendication 1, **caractérisé en ce que** le système de rétraction est réalisé sous forme modulaire, **en ce qu'**il comprend des éléments fonctionnels qui peuvent être connectés les uns aux autres par des éléments de couplage et de fermeture (3, 4), chaque élément fonctionnel présentant au moins un élément de couplage (4) ou un élément de fermeture (3) en tant que moyen de fixation.

3. Système de rétraction selon la revendication 1 ou 2, **caractérisé en ce qu'**un élément fonctionnel est réalisé au moins en partie sous forme flexible.

4. Système de rétraction selon l'une quelconque des revendications 1 à 3, **caractérisé en ce qu'**un élément rétracteur (1) est réalisé sous forme flexible.

5. Système de rétraction selon l'une quelconque des revendications 1 à 4, **caractérisé en ce que** l'élément fonctionnel flexible ou la portion flexible est élastique.

6. Système de rétraction selon les revendications 2 à 5, **caractérisé en ce que** la rétraction peut être déplacée dans le sens de son action au moyen d'un élément de fermeture (3).

7. Système de rétraction selon les revendications 2 à 6, **caractérisé en ce qu'**un élément de fermeture (3) est réalisé sous forme d'extrémité en forme de bande qui vient en prise dans un élément de couplage (4).

8. Système de rétraction selon l'une quelconque des revendications 2 à 7, **caractérisé en ce que** les éléments de couplage et de fermeture (3, 4) peuvent être déplacés les uns vers les autres de manière continue.

9. Système de rétraction selon l'une quelconque des revendications 2 à 8, **caractérisé en ce qu'**un élément de couplage (4) et/ou un élément de fermeture (3) sont réalisés sous forme de pièce séparée.

10. Système de rétraction selon la revendication 9, **caractérisé en ce qu'**un élément de fermeture (3) présente un moyen de positionnement pour le positionnement d'un élément de couplage séparé (4).

11. Système de rétraction selon l'une quelconque des revendications 2 à 10, **caractérisé en ce qu'**un élément de couplage (4) est réalisé de telle sorte qu'il retienne plusieurs éléments de fermeture (3).

12. Système de rétraction selon l'une quelconque des revendications 2 à 11, **caractérisé en ce qu'**un élément de couplage (4) est réalisé de telle sorte qu'il relie ensemble deux éléments de fermeture (3) ou un élément de fermeture (3) et un élément fonctionnel suivant un angle compris dans une plage de 0 à 180°.

13. Système de rétraction selon l'une quelconque des revendications 2 à 12, **caractérisé en ce qu'**un élément de couplage (4) présente des moyens pour l'enfichage et/ou pour l'insertion et/ou pour l'encliquetage de l'élément de fermeture.

14. Système de rétraction selon l'une quelconque des revendications 2 à 13, **caractérisé en ce qu'**un élément de fermeture (3) du système de rétraction se compose d'un matériau élastique.

15. Système de rétraction selon l'une quelconque des revendications 2 à 14, **caractérisé en ce que** la surface d'un élément de fermeture et/ou de couplage (3, 4) présente au moins en partie une microstructuration (17) amplifiant la force auto-rétentive.
